# EUROPÄISCHE PATENTSCHRIFT

(11) **EP 0 619 492 B1**
(45) Veröffentlichungstag und Bekanntmachung des Hinweises auf die Patenterteilung: **01.07.1998**
(21) Anmeldenummer: 94105238.3
(22) Anmeldetag: 05.04.1994
(51) Int. Cl.: G01N 33/52, G01N 21/01, B01L 3/00

(54) **Bestimmung eines Analyten in einer Probeflüssigkeit**
Determination of an analyte in a liquid sample
Détermination d'un analyte dans un echantillon liquide

(30) Priorität: 06.04.1993 DE 4311252
(43) Veröffentlichungstag der Anmeldung: 12.10.1994
(73) Patentinhaber: BOEHRINGER MANNHEIM GMBH, 68305 Mannheim (DE)
(72) Erfinder: Finckh, Peter, Dr., D-86911 Diessen (DE)
(74) Vertreter: Huber, Bernhard, Dipl.-Chem.

(56) Entgegenhaltungen:
- EP-A- 0 222 700
- EP-A- 0 253 581
- EP-A- 0 308 232
- WO-A-92/17769
- US-A- 4 391 906

## Beschreibung

Die vorliegende Erfindung betrifft ein Verfahren zur Bestimmung eines Analyten in einer Probeflüssigkeit in homogener flüssiger Phase durch eine in mehreren zeitlich voneinander getrennten Stufen ablaufenden Reaktion und ein zur Durchführung dieses Verfahrens geeignetes Reaktionsgefäß.

Für den spezifischen Nachweis bestimmter Parameter bzw. Analyten in der klinischen Diagnostik ist es oft notwendig, zwei oder mehrere chemische Reaktionen in unterschiedlichen, zeitlich voneinander getrennten Stufen ablaufen zu lassen. Dabei dürfen die für den zweiten oder einen weiteren Reaktionsschritt erforderlichen Reagenzien während des ersten Reaktionsschritts nicht in der Reaktionsmischung anwesend sein. Diese Vorgehensweise bedarf einer speziellen Zeitsegmentierung von Reaktionsschritten.

Eine derartige Reaktionsführung wird in der Technik bei naßchemischen Analyse-Automaten durch Zugabe der Reagenzien zu unterschiedlichen Zeitpunkten aus unterschiedlichen Vorratsflaschen gewährleistet (z.B. Hitachi-Automaten).

Weitere gängige Verfahren bestehen darin, daß die gegebenenfalls verdünnte oder vorbehandelte Probelösung nacheinander verschiedene Schichten oder Bereiche eines porösen und saugfähigen Testträgers durchwandert, wobei die verschiedenen Schichten oder Bereiche jeweils die verschiedenen Reagenzien enthalten. Die zeitliche Trennung und Steuerung der Reaktion wird hier durch die Saug- bzw. Fließgeschwindigkeit der Reaktionslösung bestimmt.

Aus der therapeutischen Galenik ist eine Vielzahl von Methoden und Rezepturen bekannt, die zu einer verzögerten Freisetzung von therapeutischen Wirkstoffen am Wirkort führen. Eine Methode zeichnet sich dadurch aus, daß man eine äußere Schicht verwendet, die nur unter ganz speziellen chemischen Bedingungen aufgelöst werden kann, die am Wirkort, aber nicht an den vorher durchlaufenen Orten angetroffen werden.

Bei einer anderen Methode benutzt man sogenannte Tablettensprengmittel. Hierbei besteht das Innere einer Tablette aus einem quellfähigen Material, das von einer weitestgehend dichten aber wasserdurchlässigen Hülle umgeben ist. Nach einer gewissen Zeit hat das Innere der Tablette soviel Wasser aufgenommen, daß der Quelldruck den Mantel sprengt und dann den im Inneren enthaltenen Wirkstoff freisetzt. Aufgrund des Auftretens größerer unlöslicher Fragmente ist dieses Prinzip jedoch bei einem Verfahren zur Bestimmung eines Analyten in einer Probelösung ungeeignet.

In einer dritten Methode wird der Wirkstoff mit einem unlöslichen, jedoch porösen, die Diffusion stark behindernden Stoff, einem mikroskopischen Netzwerk, umgeben. Auf diese Weise wird die Freisetzung des enthaltenen Wirkstoffs nicht verhindert, sondern nur mehr oder minder stark verzögert, was zu einer gleichmäßigen langsamen Wirkstoffabgabe über längere Zeiträume führt. Da bei dieser Methode größere unlösliche Bestandteile auftreten und eine zwar konstante, aber sehr kleine Abgaberate des Wirkstoffes gefunden wird, läßt sich auch dieses Verfahren nicht zur Bestimmung eines Analyten in einer Probeflüssigkeit durch eine in mehreren zeitlich voneinander getrennten Stufen ablaufenden Reaktion verwenden.

In der Technik ist bekannt, daß sich eine ganze Reihe wasserlöslicher Polymere je nach ihrer Kettenlänge bzw. der Anzahl polarer Seitengruppen mit einer mehr oder weniger hohen Geschwindigkeit in Wasser lösen können. Die Polymere gehen zwar letztlich vollständig bei gegebener Wassertemperatur und Rühreffizienz in Lösung, es werden allerdings hierfür stark unterschiedliche Zeitspannen benötigt. Beispiele für solche wasserlöslichen Polymere sind Polyvinylalkohole, Polyvinylpyrrolidone, Polyacrylsäuren und andere.

In der EP-A-0 308 232 wird ein Filtersystem beschrieben, das zum Zwecke der Verzögerung der Wasserfront eine mit einem wasserlöslichen Polymer getränkte Membran aufweist.

Die US-A-4,673 654 offenbart den Überzug eines Reagenz für einen Peroxidase-Test mit einem wasserlöslichen Polymer auf einem inerten wasserlöslichen Material zusammen mit einem zweiten Reagenz, um die beiden Reagenzien bis zur Verwendung im Test voneinander getrennt zu halten. Die Möglichkeit einer zeitlich sequenziellen Freisetzung beider Reagenzien wird nicht offenbart.

Die EP-B-0 253 581 offenbart ein analytisches Element zur Bestimmung eines Analyten in einer wäßrigen Flüssigkeit, umfassend eine erste Reagenzzone mit einem Träger und einem ersten biologisch aktiven Material und eine zweite Reagenzzone mit einem zweiten biologisch aktiven Material, das mit dem ersten biologisch aktiven Material wechselwirken kann, dadurch gekennzeichnet, daß sich die ersten und zweiten Reagenzzonen in einer einzigen Schicht in dem Element befinden und daß die zweite Reagenzzone wasserlöslich ist und ein polymeres Material enthält, das natürlicherweise in Wasser löslich ist. Dieses Element dient dazu, daß zwei Reagenzien bis zum Einsatz des Testträgers voneinander getrennt bleiben, um eine Reaktion miteinander zu vermeiden. Bei Zugabe einer wäßrigen Probeflüssigkeit löst sich das Polymer auf und das zweite Reagenz ist verfügbar. Bei diesem Element wird die schnelle Freisetzung des zweiten Reagenz aus dem wasserlös-lichen Polymer als vorteilhaft beschrieben. Eine sequenzielle Freisetzung der beiden Reagenzien zu bestimmten Zeitpunkten wird dagegen nicht offenbart. Der Aufbau des Testträgers in mehreren getrennten Zonen dient nur der Lagerstabilität. Es werden vorzugsweise solche Polymere verwendet, die sich in weniger als 120 Sekunden nach Kontakt mit der Probe auflösen.

Auch eine Verwendung organischer Lösungsmittel zum Aufbringen biochemischer Reagenzien auf Teststreifen für diagnostische Tests ist bekannt. Die US-A-4,391,906 beschreibt die Verwendung einer Tränklösung bei der Herstellung eines Teststreifens für Glucose, die unter anderem als organisches Lösungsmittel THF, sowie eine ethanolische Lösung von Polyvinylpyrrolidon enthält. Das organische Lösungsmittel dient vermutlich zur Erhöhung der Löslichkeit von Farbstoffkomponenten in der Tränklösung oder zur Verbesserung der Trocknung.

In der US-A-4,556,640 wird die Herstellung eines Teststreifens beschrieben, der sequenziell in verschiedene Tränklösungen getaucht wird, wobei eine zwischengeschaltete Trocknung stattfindet, um eine innige Vermengung der Reagenzien der unterschiedlichen Tränklösungen bis zum bestimmungsgemäßen Gebrauch zu verhindern. Eine der Tränklösungen kann in einem organischen Lösungsmittel hergestellt sein, bzw. eine Suspension aus organischer und wässriger Phase sein, um ein Wiederauflösen der Reagenzien der ersten Tränklösung bei Behandlung mit der zweiten Tränklösung zu verhindern.

Der oben genannte Stand der Technik weist jedoch erhebliche Nachteile auf. So erlauben die bekannten gebrauchsfertigen Testelemente, bei denen außer der gegebenenfalls vorverdünnten Probeflüssigkeit kein weiteres Reagenz zugegeben werden muß, keine Steuerung von mehreren Reaktionsstufen. Bei einem chromatographischen Bestimmungsverfahren, worin die Reaktionslösung nacheinander verschiedene Reaktionszonen durchwandert, wird als essentielles Element ein saugfähiges Material benötigt, das hochstrukturiert ist und daher mit den unterschiedlichen Reagenzien komplex imprägniert werden muß, wodurch ein sehr aufwendiger Herstellungsprozess bedingt ist. Auch die Vielschicht-Testelemente, bei denen die Lösung nacheinander unterschiedliche Schichten durchwandert, sind sehr komplex und damit in der Herstellung aufwendig.

Eine Anordnung, bei der ein Reagenz in einem küvettenartigen Gefäß bereits vollständig vorhanden ist, die aber die Durchführung einer in mehreren zeitlich voneinander getrennten Stufen ablaufenden Reaktion in einer homogenen flüssigen Phase ermöglicht, ist bisher nicht bekannt. Eine Aufgabe der vorliegenden Erfindung bestand somit darin, ein Verfahren zur Bestimmung eines Analyten in einer Probeflüssigkeit durch eine in mehreren zeitlich voneinander getrennten Stufen ablaufenden Reaktion zu ermöglichen, bei dem die Nachteile des Standes der Technik zumindest teilweise beseitigt sind. Insbesondere sollte ein Verfahren bereitgestellt werden, bei dem die Reaktion in homogener flüssiger Phase durchgeführt werden kann, wodurch die Verwendung eines saugfähigen Trägers mit den oben beschriebenen Nachteilen vermieden wird.

Die erfindungsgemäße Aufgabe wird durch ein Verfahren zur Bestimmung eines Analyten in einer Probeflüssigkeit durch eine in mehreren zeitlich voneinander getrennten Stufen ablaufenden Reaktion gelöst, welches dadurch gekennzeichnet ist, daß man die den Analyten enthaltende Probeflüssigkeit in ein Reaktionsgefäß gibt, das mindestens zwei zur Durchführung der Bestimmungsreaktion erforderliche Reagenzien in einer leicht in der Probeflüssigkeit löslichen und räumlich voneinander getrennten Form enthält, wobei ein für die erste Reaktionsstufe vorgesehenes erstes Reagenz in einer ohne Verzögerung in der Probeflüssigkeit löslichen Form ist, während ein für die zweite Reaktionsstufe vorgesehenes zweites Reagenz und gegebenenfalls weitere Reagenzien voneinander und von dem ersten Reagenz getrennt sind und durch Schutzschichten vor einem sofortigen Kontakt mit der Probeflüssigkeit geschützt sind, wobei die Schutzschichten derart ausgestaltet sind, daß sie einen zeitlich verzögerten Kontakt zwischen der den Analyten enthaltenden Probeflüssigkeit und dem sich unter der jeweiligen Schutzschicht befindlichen Reagenz ermöglichen, so daß eine Reaktion des Analyten mit den Reagenzien in mehreren zeitlich voneinander getrennten Stufen stattfinden kann, und daß man den Analyten nach Ablauf der Reaktion in homogener flüssiger Phase bestimmt.

Das erfindungsgemäße Verfahren findet in einem Reaktionsgefäß statt, das alle für den spezifischen Nachweis nötigen Reagenzien in trockener Form enthält. Das Reaktionsgefäß ist vorzugsweise ein küvettenartiger Testträger, der günstigerweise zur Durchführung einer optischen Bestimmung des Analyten mindestens zwei optisch transparente Seiten aufweist. Das Reaktionsgefäß ist vorzugsweise zur Aufnahme von 10 µl bis 5 ml Probeflüssigkeit, besonders bevorzugt von 20 µl bis 2 ml Probeflüssigkeit vorgesehen.

Durch das erfindungsgemäße Verfahren ist es möglich, eine zwei- oder mehrstufige Reaktion in zeitlich voneinander getrennten Teilreaktionen durchzuführen. Dabei wird eine zweites (oder ein weiteres) Reagenz in einem bestimmten Zeitraum der Reaktionslösung vorenthalten, da ansonsten eine Störung der jeweils vorangehenden Teilreaktion auftreten würde. Anschließend wird das zweite (oder weitere) Reagenz zu einem gewünschten Zeitpunkt in das flüssige Reaktionsvolumen freigesetzt, um den zweiten (oder weiteren) Reaktionsschritt zu starten. Dabei ist es zweckmäßig, wenn die Ausgestaltung der Schutzschichten oder/und die Verfahrensführung eine reproduzierbare bzw. definierbare Kontaktverzögerung des unter einer Schutzschicht befindlichen Reagenz mit der Probeflüssigkeit erlaubt.

Die Bestimmung des Analyten erfolgt bei dem erfindungsgemäßen Verfahren in homogener flüssiger Phase, d.h. die aufgelösten Reagenzien und die Probeflüssigkeit bilden ein einheitliches Reaktionsvolumen, das ansonsten gegebenenfalls nur noch nicht vollständig aufgelöste Bereiche der Schutzschichten enthält. Im Gegensatz zum Stand der Technik befindet sich die Flüssigkeit nicht in den Netzwerkfasern eines saugfähigen Trägermaterials.

Die Herstellung des für das erfindungsgemäße Verfahren geeigneten Reaktionsgefäßes erfolgt vorzugsweise dadurch, daß man zuerst ein Reagenz auf einen Teil der Innenfläche des Reaktionsgefäßes aufbringt und eintrocknet. Dann wird diese Teilfläche mit einer Schutzschicht überzogen werden. Auf entsprechende Weise können gegebenenfalls weitere Reagenzien auf die Innenfläche des Reaktionsgefäßes aufgebracht, eingetrocknet und mit weiteren Schutzschichten überzogen. Im letzten Schritt wird vorzugsweise das Reagenz der ersten Reaktionsstufe auf die Innenfläche des Reaktionsgefäßes aufgebracht und getrocknet. Dieses Reagenz muß nicht mit einer Schutzschicht überzogen werden und befindet sich daher in einer ohne Verzögerung in der Probeflüssigkeit löslichen Form.

Bei Verwendung mehrerer mit Schutzschichten überzogener Reagenzien können die einzelnen Reagenzien übereinander auf einem gleichen Teil der Innenseite des Gefäßes (und durch eine Schutzschicht voneinander getrennt) angeordnet sein oder sich jeweils auf verschiedenen Teilen der Innenseite des Gefäßes befinden. Im letzteren Fall ist es erforderlich, daß die Reagenzien durch unterschiedliche Schutzschichten vor einem Kontakt mit der Probeflüssigkeit geschützt sind, wobei dann die einzelnen Schutzschichten derart ausgestaltet sein müssen, daß sie einen Kontakt der einzelnen Reagenzien mit der Probeflüssigkeit zu dem jeweils dafür vorgesehenen Zeitpunkt ermöglichen. Hierzu können die Schutzschichten z.B. aus unterschiedlichen Materialien bestehen, andererseits ist es aber auch möglich, daß die Schutzschichten aus dem selben Material bestehen und sich nur durch ihre Dicke unterscheiden.

In einer besonders bevorzugten Ausführungsform des erfindungsgemäßen Verfahrens sind die Schutzschichten derart ausgestaltet, daß sie durch ein Einwirken der Probeflüssigkeit oder/und durch ein Einwirken äußerer physikalisch-chemischer Mittel für die Probeflüssigkeit durchlässig gemacht werden können.

So ist es beispielsweise möglich, daß die Schutzschichten bereits durch ein alleiniges Einwirken der Probeflüssigkeit für diese durchlässig gemacht werden können. Derartige Schutzschichten enthalten vorzugsweise ein wasserlösliches Polymer. Beispiele für geeignete wasserlösliche Polymere sind modifizierte Cellulosen (z.B. Carboxymethylcellulose, Carboxyethylcellulose, Carboxypropylcellulose, Hydroxyethylcellulose, Hydroxypropylcellulose), Gelatine, Polyvinylalkohole, Polyvinylpyrrolidone, Polyacrylamide, Polyacrylsäuren oder Gemische davon. Besonders bevorzugte Polymere sind Polyvinylpyrrolidon und Polyvinylalkohol.

Die Dicke der Schutzschichten hängt bei Verwendung wasserlöslicher Polymere von der erforderlichen Verzögerungsdauer, der Dicke der Reagenzschicht und der Wasserlöslichkeit des Polymers ab. Üblicherweise liegt die Dicke der Schutzschichten im Bereich von 0,1 µm bis 500 µm.

In einer weiteren Ausführungsform des erfindungsgemäßen Verfahrens sind die Schutzschichten derart ausgestaltet, daß sie nur durch zusätzliches Einwirken äußerer physikalisch-chemischer Mittel durchlässig gemacht werden können. Beispiele für derartige äußere physikalisch-chemische Mittel sind etwa die Anwendung von Wärme oder/und Strahlung. Bei dieser Ausführungsform ist es bevorzugt, daß die Schutzschicht ein photooder/und thermolabiles Polymer enthält. Beispiele für derartige photo- oder/und thermolabile Polymere sind positive Photoresiste, die aus der Produktionstechnik für elektronische Bauteile wohlbekannt sind (siehe beispielsweise Henkes, "Photochemische Verfahren in der Technik", Die Umschau 1985, Heft 2; Steppan et al., Angew. Chem. 94 (1982), 471-564; Shirai et al., Chemistry Express 3 (1988), 439-442; Shirai et al., Makromol. Chem. 190 (1989), 2099-2107 und Ito und Willson, Polymer Eng. a. Sci. 23, (1983), 1012-1017). Bekannte Photoresiste des Standes der Technik sind jedoch für das erfindungsgemäße Verfahren oft nur eingeschränkt verwendbar, da die Polymerbestandteile teilweise nur bei extremen pH-Bedingungen wasserlöslich werden oder nur eine geringe Lichtausbeute zeigen.

Beispiele für photolabile Polymere, die für das erfindungsgemäße Verfahren besonders geeignet sind, sind Triazen- oder Pentazadien-Polymere.

Derartige Polymere sind z.B. durch ein Verfahren erhältlich, wobei man eine bifunktionelle aromatische oder heteroaromatische Aminverbindung durch Diazotierung zu einem entsprechenden Bis-Diazoniumsalz umsetzt und dieses anschließend mit einer Aminogruppen-haltigen Verbindung in einer Polykondensationsreaktion zur Erzeugung eines Polymers umsetzt. Als bifunktionelle aromatische oder heteroaromatische Aminverbindungen kann man beispielsweise Bis(aminoaryl)verbindungen, vorzugsweise Bis(aminoaryl)-Verbindungen der Formel III 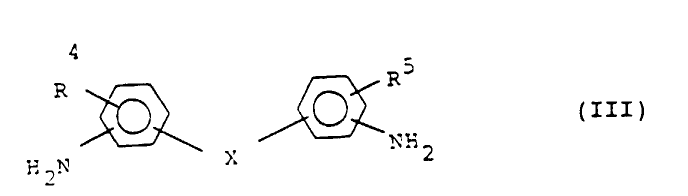 verwenden, worin X eine chemische Bindung, O, S, S-S, SO, SO₂, CO, CO-NH, CS, N=N, NH, N(Alkyl), Y, COO, SO₂-NH, NH-Y-NH, NH-CO-NH, NH-SO₂-NH oder eine gesättigte oder ungesättigte Kohlenwasserstoffgruppe bedeutet,
R⁴ und R⁵ gleich oder verschieden sind, einen oder mehrere Substituienten am aromatischen Ring darstellen und jeweils H, C₁₋₄-Alkyl, C₁₋₄-Alkoxy, Y, CN, N(Alkyl)₂, Halogen, N=N-Y, Thioether, -OY, SO₃H, CO₂H oder deren Salze bedeuten und Y eine gegebenenfalls substituierte aromatische oder nichtaromatische cyclische Kohlenwasserstoffgruppe bedeutet. Spezifische Beispiele solcher Verbindungen sind etwa Bis-(4-aminophenyl)-ether, 3,3'-Dimethoxybenzidin, 4,4'-Diaminobenzanilid, 4,4'-Diaminostilben, 4,4'-Diaminostilben-2,2'-disulfonsäure oder Bis-(4-aminophenyl)-amin oder auch monoaromatische oder heteroaromatische bifunktionelle Aminverbindungen. Als Aminogruppen-haltige Verbindung verwendet man vorzugsweise
(a) ein bifunktionelles primäres oder sekundäres Amin der allgemeinen Formel (I)

   R¹ - NH - Z - NH - R² (I),

   worin Z eine gegebenenfalls substituierte nichtaromatische Kohlenwasserstoffgruppe oder eine polymeren Rest darstellt,
   R¹ und R² gleich oder verschieden sind und jeweils Wasserstoff, eine gegebenenfalls substituierte aliphatische, ungesättigte oder/und aromatische Kohlenwasserstoffgruppe oder einen polymeren Rest darstellen, oder/und
(b) ein primäres Amin der allgemeinen Formel (II)

   H₂N - R³ (II),

   worin R³ Wasserstoff, eine gegebenenfalls substituierte aliphatische, ungesättigte oder/und aromatische Kohlenwasserstoffgruppe oder einen polymeren Rest darstellt.

Verwendet man als Aminogruppen-haltige Verbindung ein bifunktionelles primäres oder sekundäres Amin, so kann man bei Durchführung der Reaktion unter ensprechenden Bedingungen ein Triazen-Polymer erhalten. Bei Verwendung von Ammoniak oder eines primären Amins als Aminogruppen-haltige Verbindung kann man bei Wahl der geeigneten Reaktionsbedingungen Pentazadien-Polymere durch die Polykondensation erhalten.

Beispiele für bevorzugte Aminogruppen-haltige Verbindungen sind N,N'-Dimethylaminohexamethylendiamin, 2,3,5,6-Tetramethyl-p-phenylendiamin, N,N'-Dimethylaminoethylendiamin oder 2,4,6-Trimethyl-m-phenylendiamin.

Dabei ist es auch möglich, aminofunktionelle Polymere als Aminogruppen-haltige Verbindungen zu verwenden. Bevorzugte Beispiele für geeignete Polymere umfaßen aminofunktionelle Polyethylene, Poly(allyl)amine, Polyether, Polyethylenimine, Polysaccharide oder Polypeptide. Besonders bevorzugt sind aminoterminale Polyethylen- Polypropylen- oder Poly(ethylenpropylen)oxide.

Triazen- oder Pentazadien-Polymere lassen sich auch beispielsweise durch ein Verfahren herstellen, wobei man eine monofunktionelle aromatische oder heteroaromatische Aminverbindung durch Diazotierung zu einem entsprechenden Mono-Diazoniumsalz umsetzt und dieses anschließend mit einem aminofunktionellen Polymer in einer Polykondensationsreaktion umsetzt.

Durch Reaktion der Aminogruppen der Polymere mit Mono-Diazoniumsalzen können die Löslichkeitseigenschaften der Polymere in einem wäßrigen Medium dahingehend modifiziert werden, daß ein lösliches (freie Aminogruppen aufweisendes) Polymer nach partieller oder vollständiger Reaktion mit einem Diazoniumsalz unlöslich wird und sich somit zur Herstellung von Schutzschichten eignet. Bei nachfolgender Abspaltung der Triazen- oder Pentazadien-Gruppierung durch Belichtung kann das Polymer wieder in Lösung gehen, so daß die Schutzschicht aufgelöst wird.

Beispiele für monofunktionelle aromatische oder heteroaromatische Aminverbindungen sind Anilin oder mit einem oder mehreren Substituenten am Benzolring substituierte Aniline, z.B. 3,4,5-Trimethoxyanilin oder entsprechende Substanzen. Bevorzugte Beispiele für aminofunktionelle Polymere sind z.B. die bereits oben genannten Aminogruppen-haltigen Polyethylene, Poly(allyl)amine, Polyether, Polyethylenimine, Polysaccharide oder Polypeptide, insbesondere aminoterminale Polyethylen-, Polypropylen-, oder Poly-(ethylenpropylen)-oxide.

Bei Verwendung von Schutzschichten aus photolabilen Polymeren wird vorzugsweise ein Reagenz auf einen Teil der Innenfläche eines Reaktionsgefäßes, vorzugsweise eines küvettenartigen Gefäßes, aufgebracht und eingetrocknet. Danach wird diese Teilfläche und somit auch das Reagenz mit einem geeigneten positiven Photoresist überschichtet. Auf diese Weise erhält man eine wasserbeständige Schutzschicht, die nach ausreichender Belichtung mit Strahlung geeigneter Wellenlänge wasserlöslich wird und sich ohne trübe Rückstände in Wasser auflöst. Damit wird das darunter liegende Reagenz der Probeflüssigkeit zugänglich gemacht und aufgelöst. Der besondere Vorteil dieser Anordnung liegt darin, daß unabhängig von Materialeigenschaften oder komplexen Testbedingungen die Vorreaktionszeit sehr einfach in weiten Grenzen variiert werden kann, da zum gewünschten Startzeitpunkt in einer zweiten Teilreaktion der Testträger lediglich mit einer Lampe beleuchtet wird. Das sofort mit der Probeflüssigkeit in Kontakt tretende Reagenz ist auf den anderen Teilflächen des Reaktionsgefäßes aufgebracht und eingetrocknet. Ein weiterer Vorteil dieser Ausführungsform besteht darin, daß bei der Vermischung der Probeflüssigkeit mit dem ersten Reagenz keine Gefahr einer verfrühten Auflösung der Schutzschicht und damit der Kontakt der Probeflüssigkeit mit dem zweiten Reagenz möglich ist, da die Schutzschicht bis zur Belichtung völlig wasserunlöslich ist. Gegebenenfalls kann auch eine weitere geschlossene Zwischenschicht, die kein spezifisches Reagenz enthält, zwischen der das zweite Reagenz enthaltenden Grundschicht auf dem Reaktionsgefäß und der Schutzschicht aus dem positiven Photoresist angebracht werden.

Besonders bevorzugt in beiden Ausführungsformen sind Polymere, deren Auflösungskinetik nicht wie üblich linear ist, sondern sigmoiden Charakter zeigt, da sie die rückhaltende Wirkung der Schutzschicht am Anfang des Auflösungsprozesses unterstützen, am Ende des Auflösungsprozesses aber dann eine schnellere Auflösung der Schutzschicht ergeben.

Überraschenderweise lassen sich durch das erfindungsgemäße Verfahren im hohen Maße reproduzierbare Freisetzungskinetiken von durch eine Schutzschicht abgedeckten Reagenzien erreichen. Das Zeitintervall, in dem das unter der Schutzschicht befindliche Reagenz zur Verfügung steht, läßt sich in einer ersten bevorzugten Ausführungsform (wasserlösliche Polymere) einerseits durch die Schichtdicke und andererseits durch das gewählte Polymer und in einer zweiten bevorzugten Ausführungsform (positiver Photoresist) durch den Zeitpunkt der Belichtung genau bestimmen.

Besonders bevorzugt wird das erfindungsgemäße Verfahren mit gezieltem Ein- und Ausschalten eines Durchmischungsmittels kombiniert. Hierbei wird die Probeflüssigkeit nach Zugabe in das Reaktionsgefäß mit einem Durchmischungsmittel solange gemischt, bis das erste Reagenz innig mit der Probe vermengt ist, dann wird das Durchmischungsmittel bis zu dem Zeitpunkt ausgeschaltet, an dem eine Freisetzung des zweiten Reagenz gewünscht wird und anschließend wird das Durchmischungsmittel wieder bis zu einer ausreichenden Vermengung des zweiten Reagenz eingeschaltet. Diese Prozedur kann gegebenenfalls auf analoge Weise für weitere Reagenzien durchgeführt werden, die sich unter weiteren Schutzschichten befinden. Ein bevorzugtes Durchmischungsmittel insbesondere bei Reaktionsvolumen von < 1 ml ist Ultraschall. Hierbei können z.B. Schallgeber verwendet werden, die fest mit der Gefäßwand verbunden sind (siehe z.B. US-A-4,930,898 oder EP-A-0 052 322). Weiterhin kann das Mischen auch durch in die Flüssigkeit eingetauchte Rührer (magnetische Rührfische, Rührpaddel), durch Rütteln des Gefäßes oder durch Anblasen mit einem Luftstrahl (siehe z.B. US-A-4,815,978) erfolgen.

Mit Hilfe eines Durchmischungsmittels wird beispielsweise bei Verwendung wasserlöslicher Polymere eine Variation des Zeitintervalls des ersten Reaktionsschritts in weit größeren Grenzen möglich, da bei einer makroskopisch ruhenden Flüssigkeit nur die Diffusion zur Auflösung und Vermischung des in der Oberfläche angelösten Schutzfilmes aus wasserlöslichem Polymer beiträgt. Diese Diffusion ist aufgrund der hohen Zähigkeit der Polymerlösung bei hoher Konzentration, wie sie an der Oberfläche des Films zwangsläufig vorliegt, nur sehr gering und damit relativ ineffektiv. Die hohe Zähigkeit der gerade angelösten Filmoberfläche schützt gleichsam den darunterliegenden Film vor weiterer schneller Auflösung. In dieser Zeit quillt der Film jedoch an, indem er Wasser aufnimmt, so daß er im darauffolgenden zweiten Mischintervall sich wesentlich rascher auflösen und homogener vermengen läßt. Damit läßt sich eine besonders vorteilhafte, wesentlich steilere Konzentrationsstufe für den Kontakt des zweiten Reaktanden mit der Probeflüssigkeit erreichen. Dies ist dann besonders vorteilhaft, wenn die Präzision des Startzeitpunktes des zweiten Reaktionsschritts die Präzision des Analysenergebnisses mitbestimmt, wie dies z.B. bei Pseudoendpunkttesten der Fall ist.

Auch bei Durchführung des erfindungsgemäßen Verfahrens mit thermo- oder/und photolabilen Schutzschichten kann die Verwendung eines Durchmischungsmittels bevorzugt sein.

Bei Verwendung von Schutzschichten, die polymere Materialien enthalten, werden die Schutzschichten vorzugsweise durch eine mindestens teilweise Auflösung für die Probeflüssigkeit durchlässig gemacht. Besonders bevorzugt findet eine im wesentlichen vollständige Auflösung der Schutzschichten statt. Die Zeit für eine zur Freisetzung des Reagenz führende Auflösung der Schutzschichten liegt vorzugsweise im Bereich von 10 Sekunden bis 1 Stunde.

Zur Durchführung der Bestimmung des Analyten ist es weiterhin im allgemeinen zweckmäßig, daß eines der Reagenzien oder die Probeflüssigkeit eine Indikatorsubstanz enthält, durch die eine Bestimmung des Analyten ermöglicht wird. Die Bestimmung des Analyten findet vorzugsweise mit optischen Methoden (z.B. spektrometrisch) statt. Hierzu ist es bevorzugt, ein geeignetes küvettenartiges Reaktionsgefäß (z.B. ein Mikrogefäß mit ca. 5 mm Durchmesser und 2-3 mm Höhe) mit mindestens 2 optisch transparenten (d.h. zur Durchführung einer optischen Bestimmung geeigneten) Seiten zu verwenden. Es ist jedoch auch möglich, ein Reaktionsgefäß mit nur einer optisch transparenten Seite zu verwenden, wenn die Messung z.B. von oben durch den Meniskus der Flüssigkeit und den Boden des Gefäßes erfolgt.

Ein weiterer Gegenstand der vorliegenden Erfindung ist ein Reaktionsgefäß zur Bestimmung eines in einer Probeflüssigkeit vorliegenden Analyten durch eine in mehreren, zeitlich voneinander getrennten Stufen ablaufende Reaktion, dadurch gekennzeichnet, daß es mindestens zwei zur Durchführung der Bestimmungsreaktion erforderliche Reagenzien in einer leicht in der Probeflüssigkeit löslichen und räumlich voneinander getrennten Form enthält, wobei ein für die erste Reaktionsstufe vorgesehenes erstes Reagenz in einer ohne Verzögerung in der Probeflüssigkeit löslichen Form ist, während ein für die zweite Reaktionsstufe vorgesehenes zweites Reagenz und gegebenenfalls weitere Reagenzien voneinander und von dem ersten Reagenz getrennt sind und durch Schutzschichten vor einem sofortigen Kontakt mit der Probeflüssigkeit geschützt sind, wobei die Schutzschichten derart ausgestaltet sind, daß sie einen zeitlich verzögerten Kontakt zwischen der den Analyten enthaltenden Probeflüssigkeit und dem sich unter der jeweiligen Schutzschicht befindlichen Reagenz ermöglichen, so daß eine Reaktion des Analyten mit den Reagenzien in mehreren zeitlich voneinander getrennten Stufen stattfinden kann.

Die Schutzschichten im Reaktionsgefäß sind vorzugsweise derart ausgestaltet, daß sie durch ein Einwirken der Probeflüssigkeit oder/und durch ein Einwirken äußerer physikalisch-chemischer Mittel für die Probeflüssigkeit durchlässig gemacht werden können. Im übrigen sind detaillierte Ausgestaltungen der erfindungsgemäßen Reaktionsgefäße im Zusammenhang mit der Beschreibung des Verfahrens erläutert worden.

Das Auftragen von Reagenzien und Schutzschichten auf die Innenseite des erfindungsgemäßen Reaktionsgefäßes kann durch verschiedene Methoden erfolgen, beispielsweise durch Auftropfen mittels Pipette, Bedrucken mit unterschiedlichen Druckverfahren, Beschichten mittels Rakeln oder auch Gießen.

Da die verwendeten Reagenzien in der wäßrigen Probeflüssigkeit löslich sein müssen, ist es besonders vorteilhaft (wenn auch nicht unbedingt erforderlich), wenn ein die Schutzschicht bildendes Polymer aus einer organischen Lösung aufgebracht wird, in der das sich darunter befindliche Reagenz selbst nicht löslich ist. Auf diese Weise wird verhindert, daß das Reagenz durch Aufbringen der Schutzschicht teilweise angelöst wird und sich teilweise in den unteren Bereich der Schutzschicht einmischt, was zu einer weniger scharfen Freisetzungsstufe für das Reagenz führen würde.

Die Erfindung soll weiterhin durch die folgenden Beispiele und die Abbildungen 1-5 erläutert werden:
- Fig. 1: zeigt eine Auflösungskinetik wasserlöslicher Polyvinylpyrrolidon- und Polyvinylalkohol-Polymere.
- Fig. 2: zeigt die Freisetzungskinetik eines wasserlöslichen polymeren überschichteten Farbstoffes.
- Fig. 3: zeigt den Einfluß der Dicke der Schutzschicht auf die Rückhaltezeit.
- Fig. 4: zeigt eine Auflösungskinetik mit zwei diskreten Rührintervallen.
- Fig. 5: zeigt das Ergebnis eines Pankreas-α-Amylase-Tests bei Durchführung des erfindungsgemäßen Verfahrens.

### Beispiel 1:

### Auflösungskinetik unterschiedlicher wasserlöslicher Polymere

Durch Auftropfen und anschließendes Eintrocknen einer wäßrigen Lösung der entsprechenden wasserlöslichen Polymere, die als Indikator 0,2 % eines Farbstoffes (Amarant) enthielt, wurde auf dem Boden einer handelsüblichen Balbmikroabsorptions-Küvette ein runder Filmfleck hergestellt. Nach Trocknen dieses Films wurden 500 µl Wasser zugegeben und mit einem von oben eingetauchtem Spatelrührer ständig gemischt. Gleichzeitig wurde bei der für den Farbstoff geeigneten Wellenlänge (522 nm) die Extinktion als Funktion der Zeit registriert, d.h. die Konzentration des ursprünglich im Film befindlichen Farbstoffes in der Lösung wurde als Indikator für die Auflösung des Films selbst verwendet.

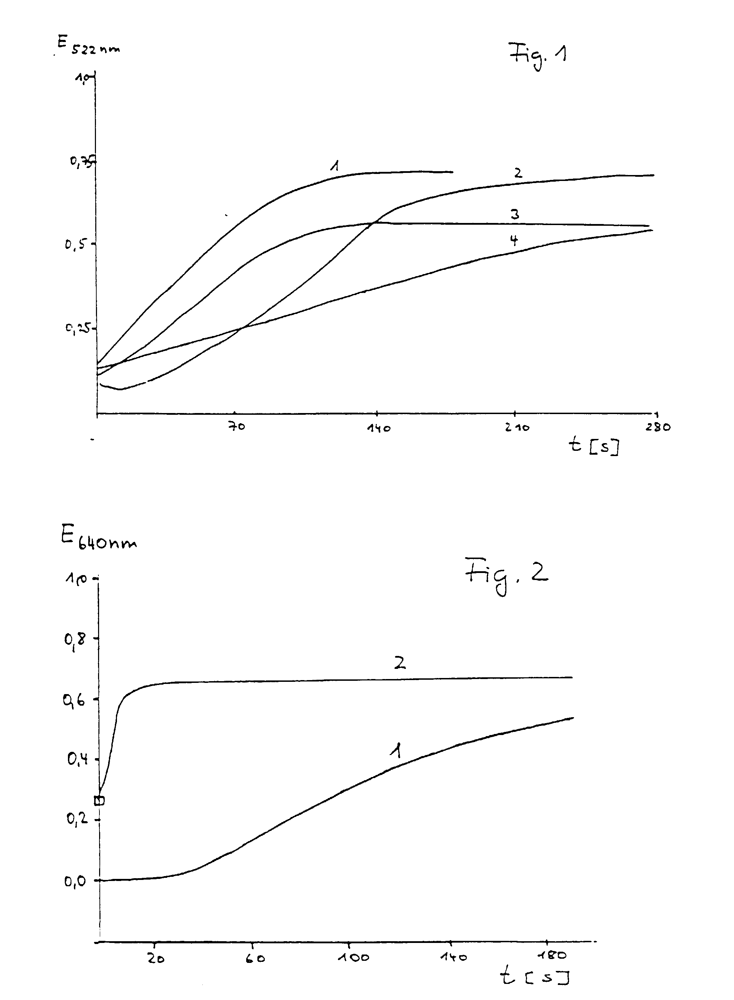

Der Versuch wurde mit unterschiedlichen Polyvinylpyrrolidon (PVP)- und Polyvinylalkohol (PVA)-Polymeren durchgeführt. Das Ergebnis ist in Fig. 1 dargestellt.

Aus Fig. 1 geht hervor, daß man durch Auswahl unterschiedlicher Polymere die Verzögerungszeit bis zur vollständigen Freisetzung in weiten Grenzen variieren kann. Besonders bemerkenswert ist die sigmoide Form der Auflösungskurve für das Polyvinylpyrrolidon PVP 360 (Kurve 2).

| | |
|---|---|
| Versuchsbedingungen: | |
| Konzentration der Polymere: | 4% PVP bzw. 5% PVA |
| Farbstoff: | Amarant 0,2 % |
| Tropfenvolumen: | 5 µl bei PVP bzw. 3 µl bei PVA |
| wäßrige Lösung: | 0,1 mol/l NaP0₄-Puffer pH 7 |
| Wellenlänge: | 522 nm |

- Kurve 1:: PVP 10 (Sigma 10 S/0001)
- Kurve 2:: PVP 360 (Sigma 28 F/0009)
- Kurve 3:: PVA 3-88 (Hoechst Mowiol 3-88)
- Kurve 4:: PVA 8-88 (Hoechst Mowiol 8-88)

### Beispiel 2:

### Freisetzungskinetik eines überschichteten Farbstoffes

Auf einer Folie wurde eine Fläche von 2 x 8 mm mit einer Lösung 1 (5% PVA 4-88 (Hoechst Mowiol 4-88), 1 % Polyethylenglykol (PEG) 400, 0,1 % Triton X-100, 1 % Patentblau in 100 mmol/l Natriumphosphatpuffer pH 7) beschichtet. Diese Lösung 1 bildete nach Eintrocknen einen in Wasser praktisch spontan löslichen Film. Anschließend wurde auf die Folie großflächig mit einer Handrakel eine gleichmäßige Schicht von 200 µm Dicke einer Lösung 2 (20 % PVP 360, 0,2 % PEG 400 in Cyclohexanol) aufgetragen, der nach nochmaligem Trocknen einen gleichmäßigen, eine Lösungsverzögerung für den Farbstoff der unteren Schicht bewirkenden Filmüberzug bildet. Aus der Folie wurden 4 x 10 mm große Felder gestanzt und auf den Boden einer Halbmikroküvette gelegt. Die Versuchsdurchführung verlief dann analog zu Beispiel 1, d.h. es erfolgte eine photometrische Messung der Farbstoffkonzentration in Lösung bei gleichzeitigem ständigen Rühren. Die Messwellenlänge war 640 nm.

Das Ergebnis dieses Versuchs ist in Fig. 2 dargestellt. Die Kurve 1 zeigt eine dreimalige Wiederholung des oben beschriebenen Experiments. Die Kurve 2 zeigt als Vergleich das Ergebnis des Experiments, wenn die Abdeckschicht aus Lösung 2 weggelassen wird. In diesem Falle findet die Auflösung dann praktisch spontan statt. Der Anstieg der Absorption zu Beginn des Experiments bei Kurve 2 ist im wesentlichen nur durch die Zeit bedingt, die ein einmaliges Vermischen des Küvettenvolumens erfordert.

### Beispiel 3:

### Einfluß der Dicke der Schutzschicht auf die Auflösungskinetik

Die Durchführung dieses Versuchs erfolgte wie in Beispiel 2 beschrieben. Es wurde lediglich der Feststoffgehalt der Lösung 2 variiert, was nach der Trocknung aufgrund der konstanten Schichtdicke beim Auftrag der Lösung zu einer entsprechenden Variation der Schichtdicke des trockenen Films führt.

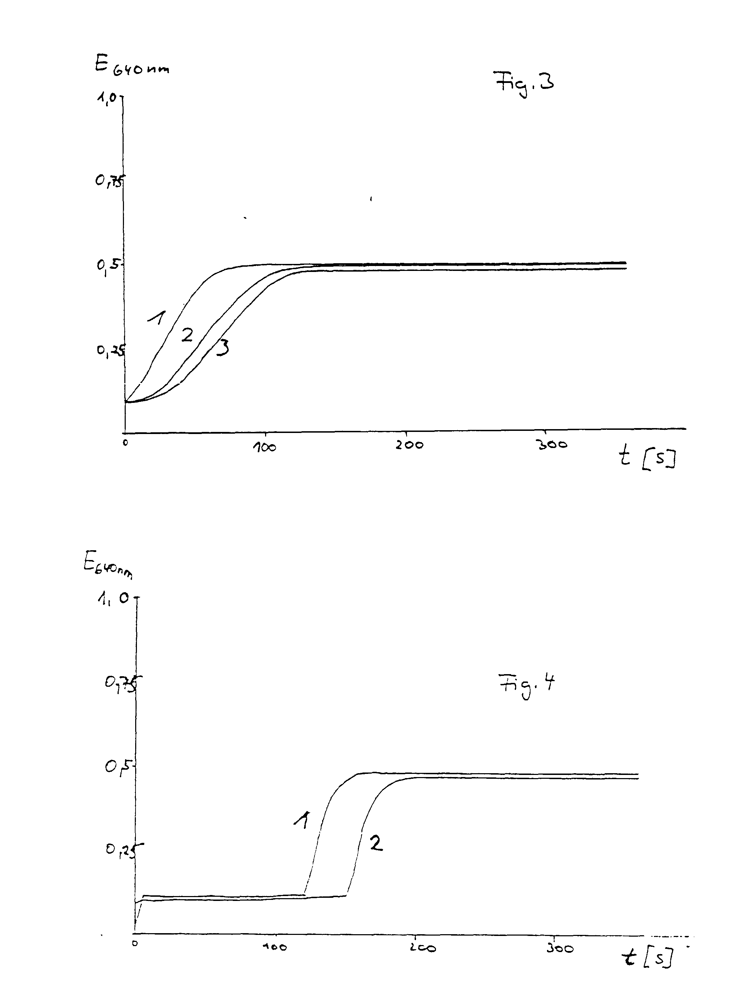

Fig. 3 zeigt deutlich, daß die Rückhaltezeit durch die Schichtdicke variiert werden kann.
- Kurve 1:: 10% PVP 360, 1% PEG 400
- Kurve 2:: 17% PVP 360, 1,7% PEG 400
- Kurve 3:: 20% PVP 360, 2% PEG 400

### Beispiel 4:

### Auflösungskinetik mit zwei diskreten Rührintervallen

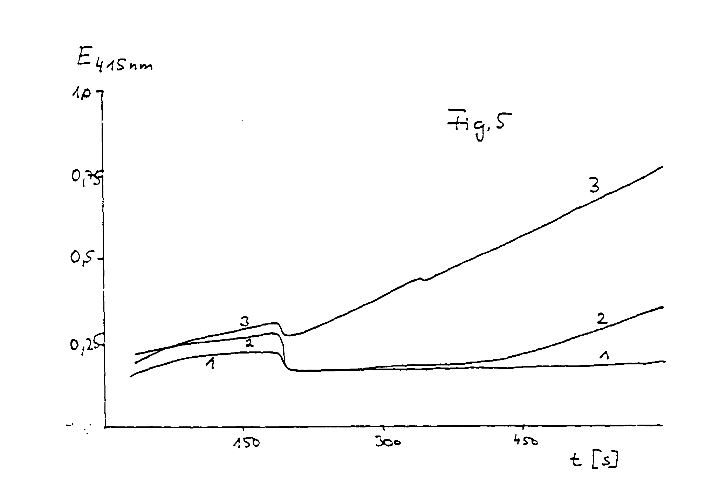

Die Durchführung des Versuchs erfolgte wie in Beispiel 3, Kurve 2, mit dem einzigen Unterschied, daß ein Rührer von der ersten bis zur fünften Sekunde eingeschaltet und dann ausgeschaltet wurde, um dann wieder ab der 125. Sekunde (Fig. 4, Kurve 1) bzw. der 155. Sekunde (Fig. 4, Kurve 2) wieder eingeschaltet zu werden. Durch Ausschalten des Rührers läßt sich die Freisetzung des Farbstoffes während des gesamten Verzögerungsintervalls praktisch vollständig verhindern, gleichzeitig wird eine deutlich sprunghaftere Freisetzung erzielt. Auf diese Weise ist es möglich, das Verzögerungsintervall in weiten Grenzen zu variieren.

### Beispiel 5:

### Pankreas-α-Amylase-Test

Es wurde ein enzymatischer Farbtest zur Bestimmung der Pankreas-α-Amylase durchgeführt (Boehringer Mannheim GmbH, Bestellnummer 1105 477). Bei diesem Test wird in einer Vorreaktion die in der Probeflüssigkeit (Serum) in etwa gleichen Anteilen enthaltene Speichel-α-Amylase durch Reaktion mit zwei spezifischen Antikörpern gehemmt. Dabei ist es kritisch, daß die Bindung dieser Antikörper nur dann effektiv, d.h. schnell stattfindet, solange nicht gleichzeitig ein α-Amylase-Substrat in der Lösung vorhanden ist, da dieses die Antikörper-Bindung kompetitiv hemmt. Daraus ergibt sich die Notwendigkeit für eine zeitlich verzögerte Zugabe bzw. Freisetzung des Substrats.

Auf den Boden des Reaktionsgefäßes (Küvette) wurde zunächst eine Lösung des Substrats (19 mmol/l 4,6-Ethyliden-G₇PNP, 105 mmol/l Hepes-Puffer pH 7,1, 52,5 mmol/l NaCl, 10,5 mmol/l MgCl₂) ) getropft und eintrocknen gelassen. Dann wurden 70 µl einer 5%igen Lösung von PVP 360 in Propanol in die Küvette gegeben und wieder eingetrocknet. Als Folge war die gesamte Innenfläche der Küvette, d.h. auch die optischen Fenster mit einem PVP-Film überzogen. Anschließend wurden die restlichen für den Testablauf notwendigen Substanzen in einer geeigneten Lösung (z.B. 29 U pro ml α-Glucosidase, 44 µg pro ml hemmende monoklonale Antikörper, 105 mmol/1 Hepes-Puffer. pH 7,1, 52,5 mmol NaCl, 10,5 mmol/l MgCl₂) anschließend wiederum auf den Boden der Küvette getropft und eingetrocknet. In die so vorbereitete Küvette wurden 70 µl vorverdünnte Probeflüssigkeit gegeben, 5 Sekunden gerührt und dann wieder von der 180. bis zur 330. Sekunde gerührt. Gleichzeitig wurde bei einer Meßwellenlänge von 415 nm die Substratumsetzung durch die α-Amylase photometrisch überwacht. Während der ersten 5 Sekunden der Reaktion wurden alle nicht von der Schutzschicht bedeckten Substanzen (α-Glucosidase, Antikörper) aufgelöst und die Vorreaktion begann. Der Signalanstieg bis zur 180. Sekunde beruhte auf einem leichten Anquellen der Schutzschicht und einer damit einhergehenden Veränderung der optischen Eigenschaften des PVP-Filmes, der sich auf den optischen Fenstern befindet (Vergleichsexperiment ohne Reagenzien). Im zweiten Mischintervall wurde der bereits angequollene Film vollständig aufgerührt, wodurch eine Lösung des Substrats erfolgte. Anschließend konnte die durch α-Amylase katalysierte Spaltung des Substrats ablaufen.

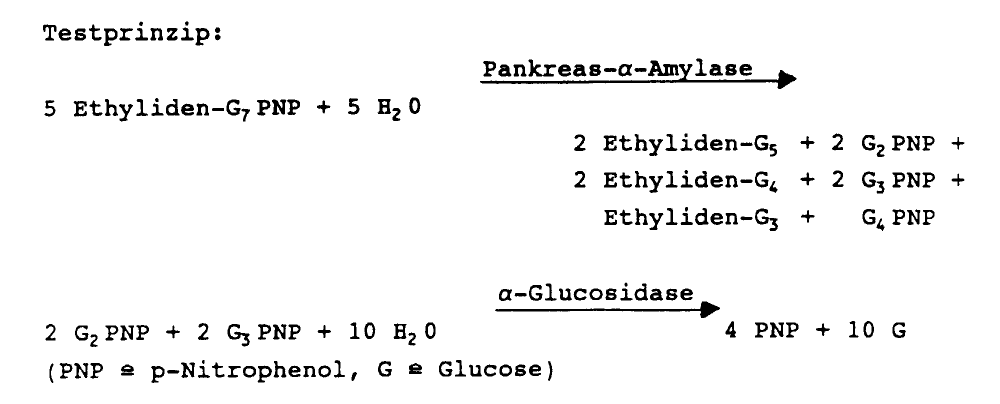

Das Ergebnis dieses Versuchs ist in Fig. 5 gezeigt.
Bei Kurve 1 wurde ein Reinpräparat Speichel-Amylase (2000 U pro l) als Probe verwendet. Man kann keinerlei Aktivität mehr beobachten. Die Hemmung durch die Antikörper war vollständig. Das Vergleichsexperiment (Kurve 2) zeigt, daß dies nicht der Fall ist, wenn in der Vorreaktionszeit eine Freisetzung des Substrats erfolgt. Dort wurden nach 360 Sekunden nämlich nochmals 2000 U/l Speichel-Amylase zugegeben, die wie aus der Abbildung ersichtlich, voll aktiv sind, da sie aufgrund der Anwesenheit des Substrats durch die Antikörper nicht mehr ausreichend gehemmt werden. Kurve 3 zeigt den Reaktionsverlauf, wenn als Probe ein Reinpräparat von Pankreas-α-Amylase (2000 U/l) verwendet wird.

## Patentansprüche

1. Verfahren zur Bestimmung eines Analyten in einer Probeflüssigkeit durch eine in mehreren zeitlich voneinander getrennten Stufen ablaufende Reaktion,
**dadurch gekennzeichnet,**
daß man die den Analyten enthaltende Probeflüssigkeit in ein Reaktionsgefäß gibt, das mindestens zwei zur Durchführung der Bestimmungsreaktion erforderliche Reagenzien in einer leicht in der Probeflüssigkeit löslichen und räumlich voneinander getrennten Form enthält, wobei ein für die erste Reaktionsstufe vorgesehenes erstes Reagenz in einer ohne Verzögerung in der Probeflüssigkeit löslichen Form ist, während ein für die zweite Reaktionsstufe vorgesehenes zweites Reagenz und gegebenenfalls weitere Reagenzien voneinander und von dem ersten Reagenz getrennt sind und durch Schutzschichten vor einem sofortigen Kontakt mit der Probeflüssigkeit geschützt sind, wobei die Schutzschichten derart ausgestaltet sind, daß sie einen zeitlich verzögerten Kontakt zwischen der den Analyten enthaltenden Probeflüssigkeit und dem sich unter der jeweiligen Schutzschicht befindlichen Reagenz ermöglichen, so daß eine Reaktion des Analyten mit den Reagenzien in mehreren zeitlich voneinander getrennten Stufen stattfinden kann, und daß man den Analyten nach Ablauf der Reaktion in homogener flüssiger Phase bestimmt.

2. Verfahren nach Anspruch 1,
**dadurch gekennzeichnet,**
daß die Schutzschichten derart ausgestaltet sind, daß sie durch ein Einwirken der Probeflüssigkeit oder/und durch ein Einwirken äußerer physikalisch-chemischer Mittel für die Probeflüssigkeit durchlässig gemacht werden können.

3. Verfahren nach Anspruch 2,
**dadurch gekennzeichnet,**
daß die Schutzschichten ein wasserlösliches Polymer enthalten.

4. Verfahren nach Anspruch 2,
**dadurch gekennzeichnet,**
daß das Einwirken äußerer physikalisch-chemischer Mittel die Anwendung von Wärme oder/und Strahlung umfaßt.

5. Verfahren nach Anspruch 4,
**dadurch gekennzeichnet,**
daß die Schutzschichten ein photo- oder/und thermolabiles Polymer enthalten.

6. Verfahren nach Anspruch 5,
**dadurch gekennzeichnet,**
daß das Polymer ein Triazen- oder ein Pentazadienpolymer ist.

7. Verfahren nach einem der Ansprüche 2 bis 6,
**dadurch gekennzeichnet,**
daß die Schutzschichten durch ein mindestens teilweises Auflösen für die Probeflüssigkeit durchlässig gemacht werden können.

8. Verfahren nach einem der Ansprüche 1 - 7,
**dadurch gekennzeichnet,**
daß man die Probeflüssigkeit nach Zugabe in das Reaktionsgefäß mit einem Durchmischungsmittel solange mischt, bis das erste Reagenz innig mit der Probe vermengt ist, das Durchmischungsmittel bis zu dem Zeitpunkt ausschaltet, an dem eine Freisetzung des zweiten Reagenzes gewünscht wird, das Durchmischungsmittel bis zu einer ausreichenden Vermengung des zweiten Reagenzes wieder einschaltet und gegebenenfalls die Prozedur analog für weitere Reagenzien durchführt.

9. Verfahren nach einem der Ansprüche 3 und 5 oder 6,
**dadurch gekennzeichnet,**
daß das die Schutzschichten bildende Polymer aus einer organischen Lösung aufgebracht wird, in der die sich darunter befindlichen Reagenzien selbst nicht löslich sind.

10. Reaktionsgefäß zur Bestimmung eines in einer Probeflüssigkeit vorliegenden Analyten durch eine in mehreren, zeitlich voneinander getrennten Stufen ablaufende Reaktion,
**dadurch gekennzeichnet,**
daß es mindestens zwei zur Durchführung der Bestimmungsreaktion erforderliche Reagenzien in einer leicht in der Probeflüssigkeit löslichen und räumlich voneinander getrennten Form enthält, wobei ein für die erste Reaktionsstufe vorgesehenes erstes Reagenz in einer ohne Verzögerung in der Probeflüssigkeit löslichen Form ist, während ein für die zweite Reaktionsstufe vorgesehenes zweites Reagenz und gegebenenfalls weitere Reagenzien voneinander und von dem ersten Reagenz getrennt sind und durch Schutzschichten vor einem sofortigen Kontakt mit der Probeflüssigkeit geschützt sind, wobei die Schutzschichten derart ausgestaltet sind, daß sie einen zeitlich verzögerten Kontakt zwischen der den Analyten enthaltenden Probeflüssigkeit und dem sich unter der jeweiligen Schutzschicht befindlichen Reagenz ermöglichen, so daß eine Reaktion des Analyten mit den Reagenzien in mehreren zeitlich voneinander getrennten Stufen stattfinden kann.

11. Reaktionsgefäß nach Anspruch 10,
**dadurch gekennzeichnet**,
daß die Schutzschichten derart ausgestaltet sind, daß sie durch ein Einwirken der Probeflüssigkeit oder/und durch ein Einwirken äußerer physikalisch-chemischer Mittel für die Probeflüssigkeit durchlässig gemacht werden können.

12. Reaktionsgefäß nach Anspruch 11,
**dadurch gekennzeichnet**,
daß die Schutzschichten ein wasserlösliches Polymer enthalten.

13. Reaktionsgefäß nach Anspruch 11,
**dadurch gekennzeichnet**,
daß das Einwirken äußerer physikalisch-chemischer Mittel die Anwendung von Wärme oder/und Strahlung umfaßt.

14. Reaktionsgefäß nach Anspruch 13,
**dadurch gekennzeichnet**,
daß die Schutzschichten ein photo- oder/und thermolabiles Polymer enthalten.

15. Reaktionsgefäß nach Anspruch 14,
**dadurch gekennzeichnet**,
daß das Polymer ein Triazen- oder ein Pentazadienpolymer ist.

16. Reaktionsgefäß nach einem der Ansprüche 10 bis 15,
**dadurch gekennzeichnet**,
daß die Schutzschichten durch ein mindestens teilweises Auflösen für die Probeflüssigkeit durchlässig gemacht werden können.

## Claims

1. Method for the determination of an analyte in a sample liquid by a reaction proceeding in several steps which are separated from one another in time,
**wherein**
the sample liquid containing the analyte is added to a reaction vessel which contains at least two reagents required to carry out the determination reaction in a form which is readily soluble in the sample liquid and spatially separated from one another wherein a first reagent intended for the first reaction step is in a form which is soluble without delay in the sample liquid while a second reagent intended for the second reaction step and optionally further reagents are separated from one another and from the first reagent and are protected from an immediate contact with the sample liquid by protective layers wherein the protective layers are designed in such a way that they enable delayed contact between the sample liquid containing the analyte and the reagent located under the respective protective layer so that a reaction of the analyte with the reagents can take place in several steps which are separated from one another in time and so that the analyte can be determined after completion of the reaction in a homogeneous liquid phase.

2. Method as claimed in claim 1,
**wherein**
the protective layers are designed in such a way that they can be made permeable to the sample liquid by the action of the sample liquid or/and by the action of external physical-chemical agents.

3. Method as claimed in claim 2,
**wherein**
the protective layers contain a water-soluble polymer.

4. Method as claimed in claim 2,
**wherein**
the action of external physical-chemical agents includes the application of heat or/and radiation.

5. Method as claimed in claim 4,
**wherein**
the protective layers contain a photolabile or/and thermolabile polymer.

6. Method as claimed in claim 5,
**wherein**
the polymer is a triazene or a pentazadiene polymer.

7. Method as claimed in one of the claims 2 to 6,
**wherein**
the protective layers can be made permeable to the sample liquid by an at least partial dissolution.

8. Method as claimed in one of the claims 1 - 7,
**wherein**
the sample liquid, after addition to the reaction vessel, is mixed with a mixing agent until the first reagent is intimately mixed with the sample, the mixing agent is switched off until the time at which a release of the second reagent is desired, the mixing agent is switched on again until the second reagent has been adequately mixed and if desired the procedure is carried out analogously for further reagents.

9. Method as claimed in one of the claims 3 and 5 or 6,
**wherein**
the polymer forming the protective layers is applied from an organic solution in which the reagents located under it are themselves not soluble.

10. Reaction vessel for the determination of an analyte present in a sample liquid by a reaction proceeding in several steps which are separated from one another in time,
**wherein**
it contains at least two reagents required to carry out the determination reaction that are in a form which is readily soluble in the sample liquid and are spatially separated from one another, wherein a first reagent intended for the first reaction step is in a form which is soluble without delay in the sample liquid, while a second reagent intended for the second reaction step and optionally further reagents are separated from one another and from the first reagent and are protected from an immediate contact with the sample liquid by protective layers wherein the protective layers are designed in such a way that they enable a delayed contact between the sample liquid containing the analyte and the reagent located under the respective protective layer so that a reaction of the analyte with the reagents can take place in several steps which are separated from one another in time.

11. Reaction vessel as claimed in claim 10,
**wherein**
the protective layers are designed in such a way that they can be made permeable to the sample liquid by the action of the sample liquid or/and by the action of external physical-chemical agents.

12. Reaction vessel as claimed in claim 11,
**wherein**
the protective layers contain a water-soluble polymer.

13. Reaction vessel as claimed in claim 11,
**wherein**
the action of external physical-chemical agents includes the application of heat or/and radiation.

14. Reaction vessel as claimed in claim 13,
**wherein**
the protective layers contain a photolabile or/and thermolabile polymer.

15. Reaction vessel as claimed in claim 14,
**wherein**
the polymer is a triazene or a pentazadiene polymer.

16. Reaction vessel as claimed in one of the claims 10 to 15,
**wherein**
the protective layers can be made permeable to the sample liquid by an at least partial dissolution.

## Revendications

1. Procédé de détermination d'un analyte dans un liquide échantillon par une réaction qui se déroule en plusieurs étapes séparées les unes des autres dans le temps, caractérisé en ce que l'on introduit le liquide échantillon contenant l'analyte dans un récipient réactionnel qui contient au moins deux réactifs nécessaires pour la conduite de la réaction de détermination sous une forme facilement soluble dans le liquide échantillon et séparés les uns des autres dans l'espace, un premier réactif prévu pour la première étape de la réaction étant sous une forme soluble sans retard dans le liquide échantillon tandis qu'un second réactif prévu pour la seconde étape de la réaction et éventuellement d'autres réactifs sont séparés les uns des autres et du premier réactif et sont protégés par des couches protectrices contre un contact immédiat avec le liquide échantillon, les couches protectrices étant agencées de telle manière qu'elles permettent un contact retardé dans le temps entre le liquide échantillon contenant l'analyte et le réactif situé sous la couche protectrice correspondante, de sorte qu'une réaction de l'analyte avec les réactifs peut avoir lieu en plusieurs étapes séparées les une des autres dans le temps et que l'on détermine l'analyte après le déroulement de la réaction en phase liquide homogène.

2. Procédé selon la revendication 1 caractérisé en ce que les couches protectrices sont agencées de telle manière qu'elles peuvent être rendues perméables au liquide échantillon par une action du liquide échantillon et/ou par une action d'agents physico-chimiques extérieurs.

3. Procédé selon la revendication 2 caractérisé en ce que les couches protectrices contiennent un polymère hydrosoluble.

4. Procédé selon la revendication 2 caractérisé en ce que l'action d'agents physico-chimiques extérieurs comprend l'utilisation de la chaleur et/ou d'un rayonnement.

5. Procédé selon la revendication 4 caractérisé en ce que les couches protectrices contiennent un polymère photo-et/ou thermolabile.

6. Procédé selon la revendication 5 caractérisé en ce que le polymère est un polymère de triazène ou de pentazadiène.

7. Procédé selon l'une des revendications 2 à 6 caractérisé en ce que les couches protectrices peuvent être rendues perméables au liquide échantillon par une dissolution au moins partielle.

8. Procédé selon l'une des revendications 1 - 7 caractérisé en ce que, après l'introduction dans le récipient réactionnel, on mélange de liquide échantillon avec un moyen de mélange jusqu'à ce que le premier réactif soit intimement mélangé avec l'échantillon, on arrête le moyen de mélange jusqu'au moment où l'on souhaite une libération du second réactif, on remet en service le moyen de mélange jusqu'à un mélange suffisant du second réactif et on met éventuellement en oeuvre le mode opératoire de manière analogue pour d'autres réactifs.

9. Procédé selon l'une des revendications 3 et 5 ou 6 caractérisé en ce que le polymère formant les couches protectrices est appliqué depuis une solution organique dans laquelle les réactifs sous-jacents ne sont eux-mêmes pas solubles.

10. Récipient réactionnel pour la détermination d'un analyte présent dans un liquide échantillon par une réaction qui se déroule en plusieurs étapes séparées les unes des autres dans le temps, caractérisé en ce qu'il contient au moins deux réactifs nécessaires pour la conduite de la réaction de détermination sous une forme facilement soluble dans le liquide échantillon et séparés les uns des autres dans l'espace, un premier réactif prévu pour la première étape de la réaction étant sous une forme soluble sans retard dans le liquide échantillon tandis qu'un second réactif prévu pour la seconde étape de la réaction et éventuellement d'autres réactifs sont séparés les uns des autres et du premier réactif et sont protégés par des couches protectrices contre un contact immédiat avec le liquide échantillon, les couches protectrices étant agencées de telle manière qu'elles permettent un contact retardé dans le temps entre le liquide échantillon contenant l'analyte et le réactif situé sous la couche protectrice correspondante, de sorte qu'une réaction de l'analyte avec les réactifs peut avoir lieu en plusieurs étapes séparées les une des autres dans le temps.

11. Récipient réactionnel selon la revendication 10 caractérisé en ce que les couches protectrices sont agencées de telle manière qu'elles peuvent être rendues perméables au liquide échantillon par une action du liquide échantillon et/ou par une action d'agents physico-chimiques extérieurs.

12. Récipient réactionnel selon la revendication 11 caractérisé en ce que les couches protectrices contiennent un polymère hydrosoluble.

13. Récipient réactionnel selon la revendication 11 caractérisé en ce que l'action d'agents physico-chimiques extérieurs comprend l'utilisation de la chaleur et/ou d'un rayonnement.

14. Récipient réactionnel selon la revendication 13 caractérisé en ce que les couches protectrices contiennent un polymère photo- et/ou thermolabile.

15. Récipient réactionnel selon la revendication 14 caractérisé en ce que le polymère est un polymère de triazène ou de pentazadiène.

16. Récipient réactionnel selon l'une des revendications 10 à 15 caractérisé en ce que les couches protectrices peuvent être rendues perméables au liquide échantillon par une dissolution au moins partielle.
